# EUROPEAN PATENT APPLICATION

(11) **EP 4 670 715 A1**
(43) Date of publication of application: **31.12.2025**
(21) Application number: 23924378.5
(22) Date of filing: 17.03.2023
(51) Int. Cl.: A61K 31/365, A61K 31/42, A61K 31/4985, A01N 43/80, A01N 43/90, A61K 9/20, A61P 33/00, A61P 33/10, A61P 33/14

(54) **PHARMACEUTICAL COMPOSITION COMPRISING FLURALANER, MOXIDECTIN AND PRAZIQUANTEL FOR TREATING PARASITIC INFESTATIONS IN SMALL ANIMALS**

(30) Priority: 24.02.2023 PE 0007242023
(71) Applicant: Agrovet Market S.A., San Luis, Lima, 15004 (PE)
(72) Inventor: DELGADO CRISPIN, Karen Melissa, San Juan de Miraflores Lima, 15056 (PE); MOLLEAPAZA AGUILAR, Erick Alfredo, Segunda etapa Lima, 15457 (PE); PORRAS NICHO, Mauricio Yvan, San Juan de Lurigancho Lima, 15416 (PE); CALDERON OJEDA, Jorge Umberto, La Molina Vieja Lima, 15012 (PE)
(74) Representative: JD&P Patent Attorneys Joanna Dargiewicz & Partners
(86) International application number: PCT/PE2023/050003
(87) International publication number: WO 2024/177520

(57) **Abstract**

The present invention is aimed at a pharmaceutical formulation or composition and a method of manufacture or production thereof, wherein the pharmaceutical composition includes fluralaner, moxidectin and praziquantel for the treatment of parasitic infestations in animals, wherein the composition may be in tablet form, more particularly as a chewable tablet and more preferably as a soft chewable tablet that manages to mask the bitter taste of the active ingredient praziquantel based on an association of bitter taste receptor blockers.

## Description

### TECHNICAL FIELD

The present invention is framed in the technical field of the pharmaceutical industry, mainly with the industry of pharmaceutical products for veterinary use.

### STATE OF THE ART

The development of pharmaceutical compositions or formulations comprising fluralaner, moxidectin and praziquantel is a current need for the treatment and control of infestations in small animals, for the treatment of parasitosis caused by flatworms and other species of tapeworms.

Document WO2022/051478 is known to report oral dosage formulations comprising an effective amount of an isoxazoline parasiticidal agent, an avermectin and a pyrazinoisoquinoline, and optionally one or more additional active ingredients, such as a tetrahydropyrimidine. The formulation may comprise fluralaner, praziquantel and moxidectin together with microcrystalline cellulose (25% to 30%), lactose monohydrate (5% to 6%), butyl hydroxytoluene (0.05-0.06%), croscarmellose sodium (3 to 5%), sodium lauryl sulfate (0.4 to 0.5%), flavoring (5 to 20%), magnesium stearate (0.5 to 1.5%) and colloidal silicon dioxide) in palatable dosage forms for veterinary use, wherein the composition has a first coated granulate comprising praziquantel and moxidectin together with a diluent, an antioxidant and a disintegrant in a physiologically acceptable polymeric matrix and a second granulate comprising an isoxazoline, mainly lotilaner together with a diluent, a wetting agent, a disintegrator and a binder.

It is also known that document WO2021/046305 reveals an appetizing granular veterinary composition comprising at least one active agent, at least one wetting agent and at least one flavoring to control or treat a condition in an animal, wherein the composition may comprise praziquantel and moxidectin in a concentration from 0.001% to 75% based on the total weight of the palatable granulated composition and may contain glycerin, fatty acids, polyethylene glycol, croscarmellose sodium and microcrystalline cellulose as a disintegrant, sodium lauryl sulfate and magnesium stearate as lubricants, hydroxypropyl methylcellulose as a binder, butyl hydroxy anisole and butyl hydroxy toluene as antioxidants and parabens as buffering agents.

It is also known that document WO2021/046296 provides a palatable, soft chewed veterinary composition comprising at least one active agent, at least one wetting agent and at least one flavoring, wherein the composition may comprise praziquantel and moxidectin and may also contain glycerin, fatty acids, polyethylene glycol, croscarmellose sodium and microcrystalline cellulose as disintegrants, sodium lauryl sulfate and magnesium stearate as lubricants, hydroxy propyl methylcellulose as a binding agent (binder), butyl hydroxy anisole and butyl hydroxy toluene as antioxidants and parabens as buffering agents.

The patent WO2020/051106 reports an appetizing hard chewable composition comprising at least one veterinarily acceptable isoxazoline, one macrocyclic lactone, one acceptable salt form of pyrantel, at least one naturally occurring animal-based flavoring, and at least one acceptable veterinary excipient. The composition is compressed into a hard tablet. Isoxazoline can be sarolaner, afoxalaner, fluralaner or lotilaner, stabilized moxidectin (macrocyclic lactone stabilized with an antioxidant) and an antiparasitic, such as praziquantel. The composition may have croscarmellose sodium (disintegrant), polyethylene glycol, sodium lauryl sulfate, hydroxypropyl methylcellulose as a stabilizer, butyl hydroxyanisole and butyl hydroxy toluene as antioxidants, magnesium stearate as a lubricant.

The patent EP2662075 relates to improvements in the oral formulations of the anthelmintic praziquantel used for the control and treatment of endoparasite infestations in warm-blooded animals, particularly in companion animals such as cats, dogs and horses, wherein the composition comprises particles of praziquantel with moxidectin with an integral coating of a lipid or a mixture of lipids that are insoluble in water and serve to mask the bitter taste of the praziquantel. The composition may include starch and purified water.

In this sense, there is still a need to provide a pharmaceutical composition or formulation that includes an association of active ingredients that can provide comprehensive protection against external parasites such as fleas, ticks and mites, as well as against internal parasites such as heartworm, gastrointestinal nematodes and tapeworms, among others. The proposed composition contains an active ingredient from the isoxazoline family, an avermectin and a synthetic derivative of isoquinoline-pyrazine.

The proposed composition is a broad-spectrum parasiticide containing an association of fluralaner, moxidectin and praziquantel available for oral administration. The composition of oral administration where the masking of the bitter taste of one of the active ingredients is achieved, in the form of a tablet, more especially in a soft chewable tablet that has a high palatability.

### BRIEF DESCRIPTION OF THE INVENTION

The present invention is aimed at a pharmaceutical formulation or composition and a method of manufacture or production thereof, wherein the pharmaceutical composition comprises fluralaner, moxidectin and praziquantel for the treatment of parasitic infestations in animals, wherein the composition may be in tablet form, more particularly the latter as a chewable tablet and more preferably as a soft chewable tablet that manages to mask the bitter taste of the active substance Praziquantel based on an association of bitter taste receptor blockers.

### DETAILED DESCRIPTION OF THE INVENTION

In a first respect, the present invention refers to a composition or pharmaceutical formulation of fluralaner associated with moxidectin and praziquantel for the treatment of parasitic infestations in small animals, wherein the composition comprises fluralaner, praziquantel, moxidectin, together with excipients pharmaceutically acceptable for oral administration.

The present invention relates to a palatable oral pharmaceutical composition or formulation of fluralaner associated with moxidectin and praziquantel for the treatment of parasitic infestations in small animals, wherein the composition comprises fluralaner, praziquantel, moxidectin, together with pharmaceutically acceptable excipients such as flavor enhancers, diluents, disintegrants, humectants, plasticizers, solvents, surfactants, flavor maskers, sweeteners, binders, lubricants, preservatives, antioxidants and mixtures thereof.

In this sense, the active ingredients can be present in a concentration between 0.1% and 40% based on the total weight of the composition. In one form of invention, the active ingredient fluralaner may be in a concentration between 5% and 30% in relation to the total weight of the final pharmaceutical composition, for oral administration it may preferably be between 10% and 15% in relation to the total weight of the final pharmaceutical composition. The active ingredient praziquantel may be present in the composition at a concentration between 0.1% and 10% in relation to the total weight of the final pharmaceutical composition, for oral administration it may be preferably between 1% and 5% in relation to the total weight of the final pharmaceutical composition. The active ingredient moxidectin is found at a concentration of 0.10% to 5% relative to the total weight of the final pharmaceutical composition, for oral administration it may be preferred at a concentration of 0.10 to 1% relative to the total weight of the final pharmaceutical composition.

In relation to the pharmaceutically acceptable excipients or components that may be in the pharmaceutical composition or formulation of the present invention, such excipients are selected from the group consisting of flavor enhancers, diluents, disintegrants, humectants, plasticizers, solvents, surfactants, flavor maskers, sweeteners, binders, lubricants, preservatives, antioxidants and mixtures thereof.

As for the flavor enhancer, it can be a flavoring or a mixture of them which include flavorings of natural origin of animal origin which can be pork, chicken or beef and/or added to flavorings of synthetic origin that provide improvement in odor, these can also be of meat flavor. The improved flavor or flavor may be present in the composition or pharmaceutical formulation of the present invention in a proportion between 5% and 30% in relation to the total weight of the final pharmaceutical composition.

In addition to the diluent or disintegrant, the pharmaceutical composition according to the present invention may also comprise a solvent, a preservative, antioxidants, and a cosolvent or diluent.

The diluent or disintegrant that may be in the pharmaceutical composition according to the present invention, may be sodium starch glycolate, sodium alginate, calcium alginate, microcrystalline cellulose, pregelatinized corn starch, corn starch, which may be in a range between 5% and 40% in relation to the total weight of the final composition. In a preferred embodiment of invention, the disintegrator may comprise in addition to starch, croscarmellose sodium in a proportion between 0.5% and 5% in relation to the total weight of the final pharmaceutical composition.,

The composition according to the present invention may further comprise humectants, such as plant-based oils such as soybean oil, sunflower oil, canola oil, and blends thereof. Oils as humectants can be in a proportion between 5% and 25% in relation to the total weight of the final composition. In one form of invention, the composition may additionally contain glycerin as a humectant in a proportion between 5% and 20% in relation to the total weight of the final composition.

The pharmaceutical composition according to the present invention comprises a forming agent or plasticizer to provide the final texture of the polyethylene glycol tablet of an adequate molecular weight, which preferably should be equal to or greater than soft, such as polyvinylpyrrolidone, hydroxypropyl methylcellulose and polyethylene glycol, preferably 1000 g/mol in the present pharmaceutical composition the plasticizer may be in a concentration between 5% and 15% based on the weight of the final pharmaceutical composition.

The pharmaceutical composition according to the present invention may also contain other solvents and diluents. In a preferred embodiment of invention, the additional diluent may be microcrystalline cellulose in a concentration between 2% and 10% relative to the total weight of the final composition and preferably 2.5% by weight based on the total weight of the final pharmaceutical composition. The additional solvent may be propylene glycol in a concentration between 1% and 10% relative to the total weight of the final composition, preferably 2.5% by weight based on the total weight of the final pharmaceutical composition.

The pharmaceutical composition according to the present invention may further comprise a surfactant, preferably an anionic surfactant which may be dodecylbenzene sulfonate, sodium dioctyl sulfosuccinate, or sodium lauryl sulfate in a concentration between 0.5% and 3.0% relative to the total weight of the final composition, more preferably 2.0% based on the total weight of the final pharmaceutical composition. In a preferred embodiment of the present invention, the surfactant is sodium lauryl sulfate.

The pharmaceutical composition according to the present invention may further comprise one or more bitter taste blockers. Bitter taste blockers can be adenosine 5'-monophosphate (AMP), thymidine 5'-monophosphate (TMP), adenosine 5'-triphosphate (ATP), monosodium glutamate, and mixtures thereof, in a concentration between 0.10% and 1.0%, more preferably 0.50%, based on the total weight of the final pharmaceutical composition.

The pharmaceutical composition according to the present invention may further comprise one or more sweeteners, such as saccharin, sorbitol, aspartame and sucralose in a concentration between 0.1% and 2% based on the total weight of the final pharmaceutical composition.

The pharmaceutical composition according to the present invention may further comprise one or more binders such as copovidone, crospovidone, povidone, carboxymethylcellulose, hydroxypropyl methylcellulose in a concentration between 0.1% and 1% based on the total weight of the final pharmaceutical composition.

The pharmaceutical composition according to the present invention may also contain one or more lubricants, such as magnesium stearate, in a concentration between 0.1% and 1.0%, preferably 0.50% based on the total weight of the final pharmaceutical composition.

The pharmaceutical composition according to the present invention may also contain one or more preservatives such as sodium sulfite, sodium metabisulfite, ethyl paraben, methyl paraben and propyl paraben, in a concentration between 0.01% and 0.10% in relation to the total weight of the final composition. In a preferred embodiment of the invention, the preservatives are methyl paraben at a concentration of 0.05% and propyl paraben at a concentration of 0.02% based on the total weight of the final pharmaceutical composition.

The pharmaceutical composition according to the present invention may additionally contain antioxidants, which may be one or more synthetically produced antioxidants, such as propylgallate (PG), terbutylhydroxyquinone (TBHQ), hydroxybutylanisole (BHA) and butylated hydroxytoluene (BHT) and may be in the pharmaceutical composition or formulation in a proportion between 0.005 and 0.05% by weight of the final formulation and preferably more than 0.04% in relation to the total weight of the final composition.

The pharmaceutical composition according to the present invention may also contain purified water as a solvent during the process of obtaining the formulation in a concentration between 1.0% and 10%, more preferably a concentration of 7.0% based on the weight of the composition before its manufacture, wherein the water evaporates during the process. In addition, the composition can include as an additional solvent ethyl alcohol (ethanol) in a concentration of 1.50% based on the weight of the composition before it is obtained, which evaporates during the process.

The pharmaceutical composition according to the present invention is for veterinary use and may be in a form that is pharmaceutically acceptable for oral administration such as a tablet, more preferably as a chewable tablet and more preferably as a soft chewable tablet of high palatability, wherein the unpleasant taste of the active substance praziquantel is masked by the presence of bitter taste receptor blockers, wherein these blockers are adenosine 5'-monophosphate and monosodium glutamate.

In a second aspect, the present invention refers to the method of production of a pharmaceutical composition comprising fluralaner, moxidectin and praziquantel for the treatment of parasitic infestations in animals, wherein the method comprises the following steps:
a) Dissolve the binder(s) with ethyl alcohol;
b) Dissolve the sweetener in purified water;
c) Mix the result from step b) with the result from step a);
d) Mix praziquantel with the diluent (microcrystalline cellulose) and bitter taste blockers;
e) Perform the wet granulation with the result of steps c) and d) and dry at 45 degrees Celsius;
f) Dissolve Moxidectin in propylene glycol as a solvent;
g) Add to step f) the preservatives and continue with the dissolution;
h) Disperse the flavor enhancer(s) in glycerin over a period of 2 minutes to 15 minutes
i) Dissolve antioxidants in moisturizer (soybean oil);
j) Mix the result of step e) with fluralaner, flavor enhancers, diluent/disintegrant (starch), disintegrant (croscarmellose sodium), surfactant, flavor blockers, sweetener, and lubricant;
k) Add the result of step h) to the result of step i) and knead;
l) Add over the result of step j), the result of step g) and glycerin;
m) Add to step result k) polyethylene glycol; kneading and
n) Perform the tableting.

In step c) the mixture is carried out until total homogenization for a period of 60 minutes, more preferably 30 minutes.

In step d) and mix with the other components for a period of 30 minutes, more preferably 15 minutes.

In step e) the drying time is carried out for a period between 6 hours and 10 hours, more preferably 8 hours.

In step f) the dissolution is carried out in a suitable container until complete dissolution for a period of 1 minute to 30 minutes, more preferably 5 minutes.

In step g) the preservatives are added and the stirring is continued until complete dissolution, for a period of 20 minutes to 60 minutes, more preferably 25 minutes.

In step h) the dispersion of the flavor enhancer(s) is carried out in a period of 2 to 15 minutes, more preferably 10 minutes.

In step i) the mixture of the antioxidants in the moisturizer is done in a suitable container and for a period of 30 minutes to 120 minutes, more preferably 60 minutes.

In step j) mix for 5 minutes and place in a kneading. Then the mixing is continued for a period of 20 minutes to 120 minutes, more preferably 40 minutes.

In step k) the mixture is kneaded for a period of 30 to 60 minutes, more preferably 45 minutes, until the solid part is completely wetted.

In step I) the stirring is continued for an additional period of time from 30 minutes to 60 minutes, more preferably 45 minutes until complete wetting/forming of a moldable mass.

In step m), in a suitable container, the excipient (propylene glycol) is heated to complete melting with constant stirring at a temperature between 60°C and 70°C and kneaded until a moldable mass is formed over a period of 30 minutes to 60 minutes, more preferably 45 minutes.

### Examples of formulation or compositions according to the invention

Examples of the declared composition are shown below in table No. 1. The solvent water and alcohol used in granulation evaporate during the process, so they are not considered within the weight of the final composition.

**Table No. 1. Examples of formulas with the declared composition**

| **Formulas Evaluated (%)** | | | | | | |
|---|---|---|---|---|---|---|
| **Component** | **A** | **B** | **C** | **D** | **E** | **F** |
| Fluralaner | 12.50% | 12.50% | 12.50% | 12.50% | 12.50% | 12.50% |
| Moxidectin | 0.10% | 0.10% | 0.10% | 0.10% | 0.10% | 0.10% |
| Praziquantel | 2.50% | 2.50% | 2.50% | 2.50% | 2.50% | 2.50% |
| Flavoring | 21.00% | 21.00% | 21.00% | 21.00% | 21.00% | 21.00% |
| Corn starch | 18.49% | 17.99% | 17.89% | 18.49% | 11.49% | 12.49% |
| Soybean Oil | 12.00% | 12.00% | 12.00% | 12.00% | 12.00% | 11.00% |
| Glycerin | 10.00% | 10.00% | 10.00% | 10.00% | 10.00% | 10.00% |
| Polyethylene glycol 3350 | 10.00% | 10.00% | 10.00% | 10.00% | 10.00% | 10.00% |
| Croscarmellose sodium | 3.00% | 3.00% | 3.00% | 3.00% | 3.00% | 3.00% |
| Microcrystalline cellulose | 2.50% | 2.50% | 2.50% | 2.50% | 7.50% | 7.50% |
| Propylene glycol | 2.50% | 2.50% | 2.50% | 2.50% | 2.50% | 2.50% |
| Sodium Lauryl Sulfate | 2.00% | 2.00% | 2.00% | 2.00% | 2.00% | 2.00% |
| Sucralose | 1.00% | 1.00% | 1.00% | 1.00% | 1.00% | 1.00% |
| Adenosine 5' monophosphate | 0.50% | - | 1.00% | 1.00% | 1.00% | 1.00% |
| Adenosine triphosphate disodium | 0.50% | 1.00% | - | 1.00% | 1.00% | 1.00% |
| Monosodium glutamate | 0.50% | 1.00% | 1.00% | 0.50% | 1.00% | 1.00% |
| Magnesium Stearate | 0.50% | 0.50% | 0.50% | 0.50% | 0.50% | 0.50% |
| Hydroxypropyl methylcellulose | 0.30% | 0.30% | 0.40% | 0.30% | 0.80% | 0.80% |
| Methyl paraben | 0.05% | 0.05% | 0.05% | 0.05% | 0.05% | 0.05% |
| Propyl paraben | 0.02% | 0.02% | 0.02% | 0.02% | 0.02% | 0.02% |
| Butyl hydroxy toluene | 0.02% | 0.02% | 0.02% | 0.02% | 0.02% | 0.02% |
| Butyl hydroxy anisole | 0.02% | 0.02% | 0.02% | 0.02% | 0.02% | 0.02% |
| Ethyl alcohol* | 2.00% | 2.00% | 1.50% | 2.00% | 3.00% | 3.00% |
| Purified water* | 3.00% | 3.00% | 2.00% | 3.00% | 4.00% | 4.00% |

| | | | | | | |
|---|---|---|---|---|---|---|
| ** **Evaporated during the manufacturing process*** | | | | | | |

### Studio Design

The palatability of the different formulas under evaluation was determined by working with 113 canines of both sexes, over 8 weeks of age, of different weights, of different breeds and clinically healthy. The tablets were presented to the animals and a score was determined for each composition according to the reaction based on the scale shown in table No. 2.

**Table No. 2. Canine Acceptance Scale**

| **Score** | **Description** |
|---|---|
| 0 | The animal must be forced to eat the tablet |
| 1 | The animal shows interest at first, but should be forced to eat the tablet |
| 2 | The animal shows interest and eats the tablet when it is placed in its snout |
| 3 | The animal smells the tablet and eats it voluntarily |

### Results

Table No. 3 shows the averages for each formula under evaluation, as well as the number of canines in which they were tested. With the exception of formula D, all formulas had an average higher than 2.6, considering them as highly palatable. Formula C had an average of 3, which places it as the most palatable formula of those evaluated. In general, most of the tablets had a good acceptance. However, D had a medium acceptance (74.19%), unlike the others that had an acceptance of more than 80%. C had 100% acceptance. When performing a Kruskal Wallis test to determine if there is a statistical difference between the groups, it was determined that there are only statistically significant differences between C and D.

**Table No. 3. Acceptance results of the evaluated formulas**

| **Formula** | **Accepted** | | **Not accepted** | | **Score** | **Number of animals treated** |
|---|---|---|---|---|---|---|
| | **Number of animals** | **%** | **Number of animals** | **%** | | |
| A | 27 | 96.4 | 1 | 3.6 | 2.82 | 28 |
| B | 26 | 86.7 | 4 | 13.3 | 2.63 | 30 |
| C | 24 | 100.0 | 0 | 0.0 | 3.00 | 24 |
| D | 23 | 74.2 | 8 | 25.8 | 2.23 | 31 |
| E | 24 | 86.1 | 4 | 14.0 | 2.67 | 28 |
| F | 26 | 92.9 | 2 | 7.1 | 2.79 | 28 |

According to the evaluation, it is evident that the combination of adenosine 5'-monophosphate with monosodium glutamate in a concentration of 2.0% is effective in blocking the bitter taste of the active ingredient praziquantel.

## Claims

1. A pharmaceutical composition for the treatment of parasitic infestations in small animals, wherein fluralaner, moxidectin and praziquantel, bitter taste receptor blockers and pharmaceutically acceptable excipients are used as active ingredients.

2. The pharmaceutical composition for the treatment of parasitic infestations in small animals in accordance with claim 1, wherein the pharmaceutically acceptable excipients are selected from the group consisting of flavor enhancers, diluents, disintegrants, humectants, plasticizers, solvents, surfactants, flavor maskers, sweeteners, binders, lubricants, preservatives, antioxidants and mixtures thereof.

3. The pharmaceutical composition for the treatment of parasitic infestations in small animals according to claim 1, wherein the active ingredients are in a concentration between 0.1% and 40% based on the total weight of the composition.

4. The pharmaceutical composition for the treatment of parasitic infestations in small animals according to claim 1, wherein bitter taste receptor blockers are selected from the group consisting of adenosine 5'-monophosphate (AMP), thymidine 5'-monophosphate (TMP), adenosine 5'-triphosphate (ATP), monosodium glutamate and mixtures thereof in a concentration between 0.10% and 1.0%.

5. The pharmaceutical composition for the treatment of parasitic infestations in small animals according to claim 1, wherein it also contains purified water as a solvent in a concentration between 1.0% and 10% based on the weight of the composition before it was obtained.

6. The pharmaceutical composition for the treatment of parasitic infestations in small animals according to claim 1, wherein it includes ethyl alcohol (ethanol) as an additional solvent in a concentration of 1.50% based on the weight of the composition before it was obtained.

7. The pharmaceutical composition for the treatment of parasitic infestations in small animals in accordance with any of the preceding claims, wherein it is in the form of a tablet.

8. The pharmaceutical composition for the treatment of parasitic infestations in small animals in accordance with claim 7, wherein the tablet is a soft chewable tablet.

9. A method of production of a pharmaceutical composition comprising fluralaner, moxidectin and praziquantel for the treatment of parasitic infestations in animals, wherein the method comprises the following steps:
a) Dissolve the binder(s) with ethyl alcohol;
b) Dissolve the sweetener in purified water;
c) Mix the result from step b) with the result from step a)a;
d) Mix praziquantel with the diluent (microcrystalline cellulose) and bitter taste blockers;
e) Perform the wet granulation with the result of steps c) and d) and dry at 45 degrees Celsius;
f) Dissolve Moxidectin in propylene glycol as a solvent;
g) Add the preservatives to step f) and continue with the dissolution;
h) Disperse the flavor enhancer(s) in glycerin over a period of 2 minutes to 15 minutes;
i) Dissolve antioxidants in moisturizer (soybean oil);
j) Mix the result of step e) with fluralaner, flavor enhancers, diluent/disintegrant (starch), disintegrant (croscarmellose sodium), surfactant, flavor blockers, sweetener, and lubricant;
k) Add the result of step h) to the result of step i) and knead;
l) Add to the result of step j), the result of step g) and glycerin;
m) Add to the result of step k) polyethylene glycol; kneading and
n) Perform the tabletting.

## Amended claims

### Amended claims under Art. 19.1 PCT

1. A pharmaceutical composition for the treatment of parasitic infestations in small animals, wherein fluralaner, moxidectin and praziquantel, bitter taste receptor blockers and pharmaceutically acceptable excipients are used as active ingredients.

2. The pharmaceutical composition for the treatment of parasitic infestations in small animals in accordance with claim 1, wherein the pharmaceutically acceptable excipients are selected from the group consisting of flavor enhancers, diluents, disintegrants, humectants, plasticizers, solvents, surfactants, flavor maskers, sweeteners, binders, lubricants, preservatives, antioxidants and mixtures thereof.

3. The pharmaceutical composition for the treatment of parasitic infestations in small animals according to claim 1, wherein the active ingredients are in a concentration between 0.1% and 40% based on the total weight of the composition.

4. The pharmaceutical composition for the treatment of parasitic infestations in small animals according to claim 1, wherein bitter taste receptor blockers are selected from the group consisting of adenosine 5'-monophosphate (AMP), thymidine 5'-monophosphate (TMP), adenosine 5'-triphosphate (ATP), monosodium glutamate and mixtures thereof in a concentration between 0.10% and 1.0%.

5. The pharmaceutical composition for the treatment of parasitic infestations in small animals according to claim 1, wherein it also contains purified water as a solvent in a concentration between 1.0% and 10% based on the weight of the composition before it was obtained.

6. The pharmaceutical composition for the treatment of parasitic infestations in small animals according to claim 1, wherein it includes ethyl alcohol (ethanol) as an additional solvent in a concentration of 1.50% based on the weight of the composition before it was obtained.

7. The pharmaceutical composition for the treatment of parasitic infestations in small animals in accordance with any of the preceding claims, wherein it is in the form of a tablet.

8. The pharmaceutical composition for the treatment of parasitic infestations in small animals in accordance with claim 7, wherein the tablet is a soft chewable tablet.

9. A method of production of a pharmaceutical composition comprising fluralaner, moxidectin and praziquantel for the treatment of parasitic infestations in animals, wherein the method comprises the following steps:
a) Dissolve the binder(s) with ethyl alcohol;
b) Dissolve the sweetener in purified water;
c) Mix the result from step b) with the result from step a);
d) Mix praziquantel with the diluent (microcrystalline cellulose) and bitter taste blockers;
e) Perform the wet granulation with the result of steps c) and d) and dry at 45 degrees Celsius;
f) Dissolve Moxidectin in propylene glycol as a solvent;
g) Add the preservatives to step f) and continue with the dissolution;
h) Disperse the flavor enhancer(s) in glycerin over a period of 2 minutes to 15 minutes;
i) Dissolve antioxidants in moisturizer (soybean oil);
j) Mix the result of step e) with fluralaner, flavor enhancers, diluent/disintegrant (starch), disintegrant (croscarmellose sodium), surfactant, flavor blockers, sweetener, and lubricant;
k) Add the result of step h) to the result of step i) and knead;
l) Add to the result of step j), the result of step g) and glycerin;
m) Add to the result of step k) polyethylene glycol; kneading and
n) Perform the tabletting.
